# EUROPEAN PATENT SPECIFICATION

(11) **EP 0 454 227 B1**
(45) Date of publication and mention of the grant of the patent: **19.01.1994**
(21) Application number: 91200917.2
(22) Date of filing: 18.04.1991
(51) Int. Cl.: C07C 273/16

(54) **Process for concentrating urea solutions under vacuum**
Verfahren zur Konzentrierung von Harnstofflösungen unter Vakuum
Procédé pour la concentration de solutions d'urée sous vide

(30) Priority: 24.04.1990 IT 2012990
(43) Date of publication of application: 30.10.1991
(73) Proprietor: SNAMPROGETTI S.p.A., I-20121 Milan (IT)
(72) Inventor: Granelli, Franco, I-20148 Milan (IT)
(74) Representative: Fusina, Gerolamo

(56) References cited:
- GB-A- 2 040 711

## Description

The present invention relates to the production of urea, and, in particular, to the end step of product concentration of such a process, in which urea solution is concentrated until a practically pure, molten product is obtained, which is suitable for obtaining the solid granular, i.e., the "prilled" product.

All of the industrial processes presently used for producing urea are based on the direct synthesis of urea from ammonia and carbon dioxide, according to the overall reaction:

2 NH₃ + CO₂ = CO(NH₂)₂ + H₂O

leading to the formation of an aqueous solution of urea at a concentration of round 75% by weight.

In reality, the process is much more complex, in that it is constituted by a plurality of equilibrium reaction following each other, which require separations and recycles in order to achieve yields very close to the total conversion of the reactants according to the above cited overall reaction.

The solution obtained from the industrial facilities contains still small amounts of ammonia and of carbon dioxide which have not been converted into urea, and which have not been removed and recycled to the intermediate steps of the process.

In its several uses, urea is essentially required as a solid product, consisting of substantially spherical granules of variable sizes, according to the use the product is destined to, obtained by prilling or granulation (pellets).

These treatments have to be fed with molten urea at a concentration of 99.5-99.8%. by weight in the case of prilling, and at a concentration which may be even lower, in the case of granulation treatments.

According to the conventional processes, such an concentration step is carried out inside vacuum evaporators in which the solution at about 75% of urea is concentrated until molten urea at 99.5-99.8% is obtained.

In the most recent processes, the requirements of energy saving caused the end concentration step to be subdivided into two steps carried out under decreasing pressure: the first step is carried out under sub-atmospheric pressure (indicatively of 0.25-0.5 abs.bar) up to concentrations of the product which are higher than 90% by weight, and indicatively of 92-96% by weight, without a substantial energy consumption for vacuum production, whilst the second step, which increases the product concentration up to 99.5-99.8% by weight of urea, is carried out under high vacuum, with energy being consumed only in order to remove the last portion of evaporated water.

The operations of concentration have to be carried out at temperatures even higher than urea solidification temperature, in particular when water content of urea is so low as not to perform any longer any substantial solubilizing effects.

During the operations of end concentration, and in particular in the end concentrator of the double-step concentration processes, a phenomenon of fouling of the upper portion of the concentrator, concerned by the separated gas phase, takes place.

This fouling leads to incrustations which tend to accumulate with time and calls for such incrustations to be periodically removed in order not to compromise the quality of the product, and the efficiency of the facility. This programmed servicing prevents the accidental stops due to the falling down of the poorly soluble fouling pieces -- which might cause duct cloggings and difficulties in product discharge.

The phenomenon of the formation of such incrustations is very complex and proceeds according to rather unknown reactions and kinetics. It is anyway to be attributed to the fact that during the end concentration step, in the gas phase which develops inside the concentrator, besides water vapour residual amounts of ammonia, carbon dioxide and still other volatile components arising from urea decomposition are contained together with entrained suspended particles of molten urea, the amount which is small, but not negligible over time.

The formation of the incrustations in the upper portion of the concentrator, concerned by the gas phase developed, is attributed to these compounds contained in said gas phase. In fact, the incrustations result to be formed, besides urea and biuret, which are soluble in water, also by longer-chain compounds, which are insoluble in water, such as triuret and its higher homologous products, which are formed by urea condensation, as well as other compounds, such as cyanuric acid.

Those reactions are thought to take place on urea particles deposited on the top walls of the concentrator, and to be favoured by low pressure and relatively high temperature.

Such phenomena are quantitatively negligible as regards the process yields, in that they proceed very slowly, but in the long term they are the cause of the incrustations which are deposited on the walls of the upper portion of the concentrators. They are difficult to be removed and to be disposed off, in that they are practically useless.

The cited compounds display high melting points than urea (132°C): biuret melts at 190°C and triuret melts at 231°C. Their solubility in water is very low, and in order to remove the incrustations only mechanical cleaning remains, which should be carried out during the stops of the facility.

The process according to the present invention is defined in claim 1. It consists of a process for concentrating under vacuum urea solutions which already are at a high concentration, until molten urea at 99.5-99.8% of urea is obtained, without any substantial formation of insoluble incrustations taking place in concentration equipment. According to the present invention, the process consists in carrying out the end vacuum concentration step by injecting inside the overhead portion of the concentrator concerned by the development of the gas mixture, water vapour which expands and overheats, with the composition and the weight ratio between the gas phase and produced molten urea being thus varied.

Such an injection of overheated water vapour can be carried out either continuously or intermittently, with amounts of water vapour being fed, which are comprised within the range of from 10% to 100% by weight, and preferably comprised within the range of from 20% to 50%, relative to the weight of the vapour phase evolved during the concentration step.

In order to illustrate the characteristics and advantages of the present invention, the process is disclosed in the following by referring to a typical form of practically embodiment thereof, reported in the hereto attached figure for merely illustrative, non-limitative purposes.

The urea solution, already concentrated in the atmospheric concentration step up to a purity of 92-96%, is fed by the pipe 1 to the heat exchanger 2 of tube-bundle type, wherein it is heated to a temperature substantially higher than urea crystallization temperature, of 134-144°C in the average.

The pressure inside the heat exchanger 2 is kept comprised within the range of from 0.02 to 0.1 abs. bar, by the effect of vacuum generators, typically multi-step steam ejectors, connected which the subsequent separator vessel.

Inside the tube bundle of the heat exchanger 2 two phases are formed: a liquid phase, substantially constituted by molten urea at 99.5-99.8% by weight of urea, and a dispersed gas phase, constituted by water vapour and a small portion of ammonia, carbon dioxide and still other volatile compounds deriving from urea decomposition. The necessary heat is supplied as steam fed to the jacket side of the heat exchanger through the pipe 3, and dischared as condensate through the pipe 4.

The mixed gas-liquid phase generated inside the tube bundle of the heat exchanger 2 is fed through the pipe 5 to the separator 6 of cylindrical shape, in which the separation of the two phases takes place. According to a preferred form of practical embodiment of the present invention, the gas phase is fed tangentially to the internal cylindrical surface of the separator, such as to take advantage of the centrifugal effect caused by the difference in density of the two phases and to cause the separation of the gas phase to develop in centripetal direction -- typical of cyclone separator -- with a relative speed between the two phases being obtained which is much smaller than one might expect to obtain by computing it on the basis of the surface-area of the cross section of the separator, and with the effects of purely dynamic entraining of suspended urea particles -- which result to be a determining factor of fouling -- being hence reduced. The bottom portion of the separator 6 is made with an acute-angle conical bottom, such as to limit the stay time of molten urea, and the entraining of suspended urea particles upwards. From the bottom of the separator 6 molten urea is sent by means of the pipe 7 to the step of solidification in particulate form, prilling or granulation.

On the contrary, the separated gas phase is laterally drawn from the upper portion of the separator 6 by means of the pipe 8 and is sent to a conventional vacuum-condensation unit not shown in figure. Typically, it can be constituted by a plurality of vapour ejectors installed in cascade, with the relevant condensers.

The portion of the separator 6 which is concerned by fouling is in general the top spherical ceiling and its adjacent cylindrical walls. According to the present invention, overheated water vapour is injected into the upper portion of the separator 6 by means of the pipe 9 and is distributed by means of a plurality of nozzles which direct their jets towards the walls of the upper spherical ceiling and the cylindrical walls adjacent to it. According to a typical form of practical embodiment of the present invention, the nozzles 10 are installed on a toroidal distributor 11, so as to inject the vapour both axially and radially, with a region being established, which is protected from the above said fouling.

The number of the nozzles can be comprised within the range of from 6 to 24, and preferably of from 8 to 16, and can also be subdivided on a plurality of distributors.

The supply of water vapour through the pipe 9 is controlled by the valve 12, and can take place continuously or intermittently with the aid of a timer 13. In fact, the present Applicant was able to find that the action of protection against fouling is effective even if water vapour is only intermittently injected into the separator vessel 6.

In principle, the intermittent injection is easier to be applied in case already existing facilities have to be modified, in that the available capacity of the already existing vacuum condensation units and vacuum generators has to be taken into due account, whilst in case of new facilities the continuous injection -- which does not generate oscillations in the performance of vacuum generation units and of the condensers installed downstream the separator 6 -- is more easily applied.

### Example

In an already existing facility for urea production of rated production capacity of 1000 tons daily, the flow rate of urea solution fed to end concentration is of:
* Urea 41,717 kg/h
* Ammonia 9 kg/h
* Carbon dioxide 4 kg/h
* Water 2,650 kg/h

The concentration step is carried out at the temperature of 138°C and under a pressure of 0.03 abs.bar, and originates a molten mass of urea containing 41,620 kg/h of urea and 104 kg/h of water; a gas phase developed, which contains:
* Decomposed and entrained urea 97 kg/h
* Ammonia 9 kg/h
* Carbon dioxide 4 kg/h
* Water 2,546 kg/h

In this concentration section, a considerable fouling took place in the high portion of the separator. The incrustations were removed by water washing every three weeks and mechanical cleaning every year.

The facility was modified in order to inject water vapour by means of a toroidal distributor equipped with 8 nozzles to the overhead portion of the separator 6, as shown in the hereto attached Figure.

During an approximately 1-year long production time, during the time interval between two annual servicing operations, water vapour at 150°C was fed at a flow rate of 450 kg/h. Water vapour feed took place intermittently, i.e., every two hours and each time for about 10 minutes, with the aid of the timer 13.

After a 1-year run, at the subsequent servicing step the fouling resulted to have been reduced by about 90% as compared to the preceding runs, carried out with no water vapour injection.

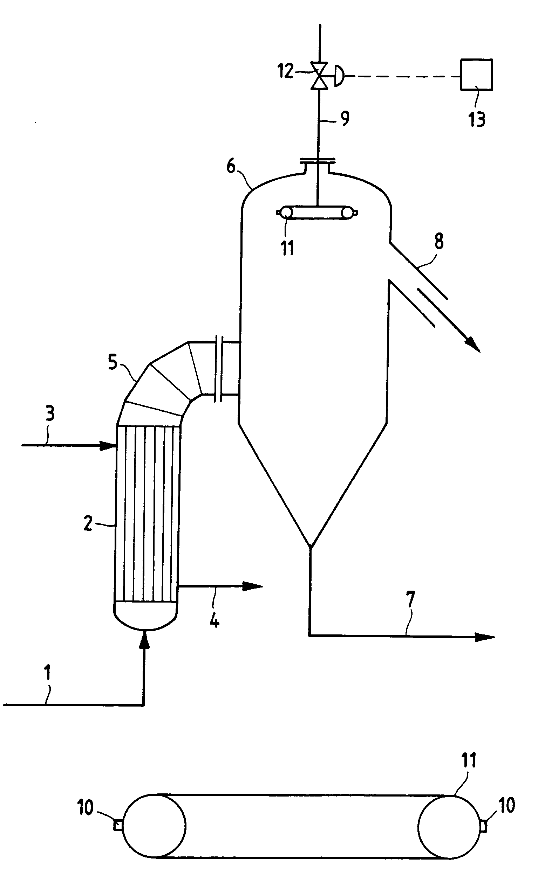

## Claims

1. Process for concentrating under vacuum urea solutions until molten urea at 99.5- 99.8 % by weight of urea is obtained, which has to be fed to the end granulation or prilling step, which process is carried out at temperatures comprised within the range of from 134 to 144°C and under pressures comprised within the range of 0,02 to 0.1 abs.bar in a concentration unit constituted by a heat exchanger 2, a separator 6 and an unit of condensation under vacuum, characterized in that in the gas phase, which develops in the separator 6 , overheated water vapour is fed in an amount comprised within the range of from 10 to 100% and preferably comprised within the range of from 20 to 50%, relative to the weight of the same gas phase and at temperatures comprised within the range of from 140 to 160°C, and preferably of about 150°C, with said overheated water vapour being fed by means of distributor nozzles so arranged that the injected water vapour will concern the overhead portion of the separator 6 and will generate a region protected from fouling.

2. Process according to claim 1, characterized in that the feed of water vapour is carried out in continuous mode.

3. Process according to claim 1, characterized in that the feed of water vapour is carried out in intermittent mode.

4. Process according to claim 1, characterized in that said overheated water vapour is distributed by means of a plurality of nozzles 10, provided in a number comprised within the range of from 6 to 24, and preferably comprised within the range of from 8 to 16, arranged on one or more distributor. 11 installed in the overhead room of the separator 6 in the nearby of its top ceiling.

## Patentansprüche

1. Verfahren zum Konzentrieren von Harnstofflösungen unter Vakuum, bis geschmolzener Harnstoff mit 99,5 bis 99,8 Gew.-% Harnstoff erhalten wird, der zu der Endgranulierungs- oder Prillierungsstufe zugeführt werden soll, welches Verfahren bei Temperaturen im Bereich von 134 bis 144°C und unter Drücken im Bereich von 0,02 bis 0,1 bar absolut in einer Konzentrierungsanlage ausgeführt wird, die aus einem Wärmetauscher 2, einer Trennvorrichtung 6 und einer Vakuumkondensationseinheit besteht, dadurch gekennzeichnet, daß in die Gasphase, die sich in der Trennvorrichtung 6 ausbildet, überhitzter Wasserdampf in einer Menge im Bereich von 10 bis 100 % und vorzugsweise im Bereich von 20 bis 50 %, bezogen auf das Gewicht dieser Gasphase, bei Temperaturen im Bereich von 140 bis 160°C und vorzugsweise bei etwa 150°C eingeführt wird, wobei dieser überhitzte Wasserdampf mit Hilfe derart angeordneter Verteilungsdüsen zugeführt wird, daß der injizierte Wasserdampf den Kopfraum der Trennvorrichtung 6 beaufschlagen und einen vor Fouling geschützten Bereich ausbilden wird.

2. Verfahren nach Anspruch 1, dadurch gekennzeichnet, daß die Wasserdampfeinspeisung in kontinuierlicher Weise vorgenommen wird.

3. Verfahren nach Anspruch 1, dadurch gekennzeichnet, daß die Wasserdampfeinspeisung in intermittierender Weise ausgeführt wird.

4. Verfahren nach Anspruch 1, dadurch gekennzeichnet, daß der überhitzte Wasserdampf mittels mehrerer Düsen 10 verteilt wird, welche Düsen in einer Anzahl im Bereich von 6 bis 24 und vorzugsweise im Bereich von 8 bis 16 vorgesehen sind und an einem oder an mehreren Verteilern 11 angeordnet sind, der bzw. die im Kopfraum der Trennvorrichtung 6 nahe ihrer Decke installiert ist bzw. sind.

## Revendications

1. Procédé pour concentrer sous vide des solutions d'urée jusqu'à ce que l'on obtienne de l'urée fondue, en un pourcentage de 99,5-99,8% en poids, qui doit être envoyée à l'étape finale de granulation ou étape de "prilling", ce procédé étant exécuté à des températures comprises dans l'intervalle allant de 134 à 144°C et sous des pressions comprises dans l'intervalle allant de 0,02 à 0,1 bar (pression absolue) dans une unité de concentration constituée par un échangeur de chaleur (2) un séparateur (6) et une unité de condensation sous vide, caractérisé en ce que dans la phase gazeuse, qui se forme dans le séparateur (6), de la vapeur d'eau surchauffée est amenée en une quantité comprise dans l'intervalle allant de 10 à 100% et, de préférence, dans l'intervalle allant de 20 à 50%, par rapport au poids de la même phase gazeuse et à des températures comprises dans l'intervalle allant de 140 à 160°C, et de préférence d'environ 150°C, ladite vapeur d'eau surchauffée étant amenée au moyen d'injecteurs de distributeur disposés de manière que la vapeur d'eau injectée concerne la partie haute du séparateur (6) et engendre une région protégée contre les souillures.

2. Procédé selon la revendication 1, caractérisé en ce que l'amenée de vapeur d'eau est effectuée suivant un mode continu.

3. Procédé selon la revendication 1, caractérisé en ce que l'amenée de vapeur d'eau est effectuée suivant un mode intermittent.

4. Procédé suivant la revendication 1, caractérisé en ce que la vapeur d'eau surchauffée est distribuée au moyen d'une pluralité d'injecteurs 10, prévus en un nombre compris dans l'intervalle allant de 6 à 24, et de préférence compris dans l'intervalle allant de 8 à 16, disposés sur l'un ou sur plusieurs distributeurs 11 installés dans l'espace supérieur du séparateur 6 au voisinage de sa paroi supérieure.
